(19) Europäisches Patentamt / European Patent Office / Office européen des brevets

(11) **EP 4 062 949 A1**

(12) **EUROPEAN PATENT APPLICATION**

(43) Date of publication:
**28.09.2022  Bulletin 2022/39**

(21) Application number: **21164631.0**

(22) Date of filing: **24.03.2021**

(51) International Patent Classification (IPC):
***A61L 15/46*** *(2006.01)*

(52) Cooperative Patent Classification (CPC):
**A61L 15/46;** A61L 2300/104; A61L 2300/404;
A61L 2300/606; A61L 2300/802

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Mölnlycke Health Care AB
402 52 Göteborg (SE)**

(72) Inventor: **CARLSSON, Erik
42941 Särö (SE)**

(74) Representative: **Tostmann, Holger Carl
Wallinger Ricker Schlotter Tostmann
Patent- und Rechtsanwälte Partnerschaft mbB
Zweibrückenstrasse 5-7
80331 München (DE)**

(54) **ABSORBENT ANTIMICROBIAL WOUND DRESSINGS WITH IMPROVED PROPERTIES AND REDUCED DISCOLORATION**

(57)    The present invention relates to fiber materials with improved properties and a process for producing the same. In particular, the present invention provides fiber materials having, at the same time, improved mechanical and antimicrobial properties as well as improved wear properties (i.e. "softness"), without the occurrence of severe discoloration imparted by the antimicrobial agent added.

Figure 2

EP 4 062 949 A1

## Description

### FIELD OF THE INVENTION

The present invention relates to fiber materials with improved properties and a process for producing the same. In particular, the present invention provides fiber materials having, at the same time, improved mechanical and antimicrobial properties as well as improved wear properties (i.e. "softness"), without the occurrence of severe discoloration imparted by the antimicrobial agent added.

These and other improvements are achieved, among others, by providing a substrate that comprises or consists of absorbent fibers that has been treated in a specific manner with an agent having at least one, preferably at least two, groups capable of forming a hydrogen bond (in the following also referred to as an *"agent"* or an *"agent capable of forming a hydrogen bond"*) and an antimicrobial coating composition comprising at least one silver-containing agent and at least one polymer.

While the fiber materials according to the present invention may be used in various fields, in particular as carrier materials, household products, hygiene products and the like, the fiber materials are of particular use in wound treatment, e.g. in wound dressings.

### BACKGROUND

Fiber materials, in particular fiber materials that are capable to absorb and retain a liquid, are useful in a variety of applications, including as carrier substrates, household products, hygiene products and in wound treatment. The area of wound treatment poses particular challenges to fiber materials, since not only is adequate fluid management required, but also integrity of the overall product containing the fiber, in particular during application, use and removal. The fiber material, in particular if part of an overall wound care product, must also be sterile, safe to use in contact with bodily fluids and, ideally, comfortable to wear for the patient.

In particular, antimicrobially-equipped wound care products are highly sought after for preventing infections and accelerate the healing of wounds, which usually requires the addition of one or more antimicrobial agents to a substrate material. Silver-containing agents are particularly suitable as antimicrobial agents. Further particularly, wound care products should not show any (severe) discoloration of the material in contact with wound, in particular any fiber material, such that, e.g., colored contaminants can easily and readily be identified on the wound care product.

WO 2018/137979 discloses fiber materials with improved mechanical properties and improved patient comfort. The fiber materials are produced by contacting a substrate material with an agent capable of forming a hydrogen bond.

WO 2017/016974 discloses a method for producing antimicrobial wound dressings containing a water-soluble antimicrobial agent. The method of WO 2017/016974 addresses the problem that certain antimicrobial agents are only soluble in highly aqueous solutions, while some materials for the production of absorbent wound dressings are incompatible with highly aqueous solutions because they gel in the presence of water. In order to address that issue, WO 2017/016974 provides a coating system comprising an antimicrobial agent, a non-aqueous solvent and one or more polymers which are believed to aid the dispersion of the antimicrobial agent and avoid settling thereof in the non-aqueous solvent.

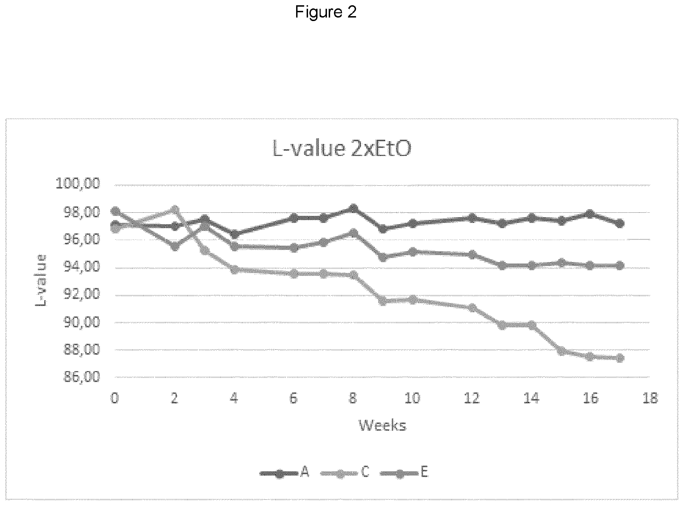

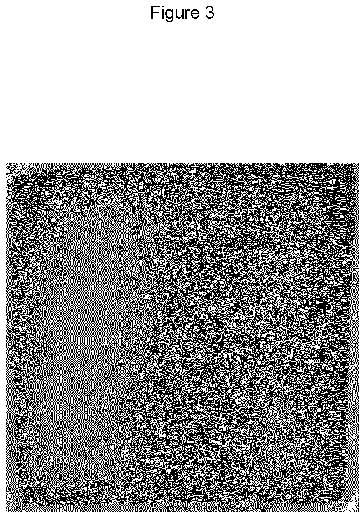

However, although the above technologies are known, it has yet not been possible to provide silver-containing fiber materials that have, at the same time, beneficial antimicrobial properties and improved mechanical and wear properties (in the meaning of "softness") without, in particular, the occurrence of severe discoloration. The reason is that - as it has been found by the inventors of the present application - a *combination* of a silver-containing antimicrobial coating composition that provides for antimicrobial properties and overcomes the problem of gelation in the presence of highly aqueous solvents (e.g. a coating composition known from WO 2017/016974) with an agent capable of providing improved mechanical and wear properties (i.e. "softness") (e.g. an agent capable of forming a hydrogen bond known from WO 2018/137979), leads to a severe discoloration. An example of such a discolored dressing is shown in Figure 3. "Discoloration" in the meaning of the present invention includes the "yellowing" of an originally "white" dressing. Such a perceived color change from "white" to "yellow" may be quantitatively captured in the CIELAB color space (L*a*b), here according to the standard ASTM D2244-16 as approved on July 1, 2016 (see Figure 2 and the discussion of ∆L values below)

"Discoloration" in the meaning of the present invention also includes the appearance of spots or freckles, typically brown, black or grey, due to silver deposition, on an otherwise essentially white or light yellow dressing (see Figure 3). The occurrence of such spots of freckles may be quantitatively described as the number of spots or freckles visible to the naked eye per cm2, as averaged over 100 such area segments defined on a 10cm times 10cm sample (as shown in Figure 3).

Discolored products, in particular significantly discolored products generally are unsuitable for practical uses, in particular for uses in wound treatment where the identification of potential (colored) contaminants on the wound care product may be of great importance. Also, there exists a natural hesitation to contact a wound with a significantly discolored wound dressing as discolored wound dressings give the impression of being "dirty" and "unhygienic". That is in particular the case for non-uniformly discolored wound dressings (e.g. wound dressing having spots and stains). While uniformly discolored wound dressings (e.g. wound dressing that have a uniform grey color) are still accepted to a certain extent, wound dressing having non-uniform discolorations (e.g. wound dressing having spots, "speckles" and stains) are virtually entirely unaccepted because they are generally considered to be "dirty" and "unhygienic".

**SUMMARY OF THE INVENTION**

In view of the above drawbacks, it is an object of the present invention to provide fiber materials (in particular fiber materials for wound therapy, in particular fiber materials comprised in a wound dressing) exhibiting, at the same time, improved mechanical and antimicrobial properties as well as improved wear properties without the occurrence of severe discolorations, ideally without any significant discoloration, in particular without the occurrence of non-uniform discolorations such as spots and stains.

The inventors of the present application have surprisingly found that significant discoloration may be observed if a substrate comprising or consisting of absorbent fibers (as are often used in wound dressings) is coated with a coating composition comprising a silver-containing antimicrobial agent, a polymer for dispersing the silver-containing antimicrobial agent (e.g. HPC), and an agent capable of forming hydrogen bonds (e.g. glycerol), when all components are added in one step and when the specifically claimed sequence of adding these components is not observed. Thus, the inventors of the present application have found that an agent capable of forming hydrogen bonds (e.g. glycerol) cannot simply be added to a silver-containing antimicrobial coating composition known from WO 2017/016974.

The inventors of the present application have found that this drawback can be avoided by using a process, in which the substrate is **first** contacted with an agent capable of forming a hydrogen bond (e.g. glycerol) and **then,** in a further step, which is performed **separate from** and **after** the first step, the obtained substrate is contacted with a silver-containing antimicrobial coating composition (e.g. silver salts in a protic solvent and in the presence of a polymer) that is configured to avoid gelation of the substrate.

In a **_first aspect,_** the present invention thus relates to a process for making a modified fiber material, wherein the process comprises the following steps:

> (1) providing a substrate comprising or consisting of absorbent fibers;
> (2) contacting the substrate with an agent having at least one, preferably at least two, groups capable of forming a hydrogen bond;
> (3) contacting the product of step (2) with an antimicrobial coating composition comprising at least one silver-containing antimicrobial agent and one or more polymers in a solvent system that comprises a non-aqueous solvent.
> (4) drying the product of of step (2) or the product of step (3), or both.

In a **_second aspect,_** the present invention relates to a fiber material, in particular a wound dressing, obtained or obtainable by the process of the first aspect.

It is noted that the fiber material, in particular wound dressing as obtained or obtainable by the process of the first aspect is a novel material as such (i.e. not by virtue of the process by which it is made) since the fiber material, in particular wound dressing does not show, or does at least not show to the same extent, discoloration as described above, in particular after a "curing/ageing" process as described below, compared to an otherwise same fiber material, in particular wound dressing, in which the sequence of steps as described above in regard to the first aspect has not been followed, for example because contacting steps (2) and (3) as described above have been implemented concomitantly.

In a **_third aspect,_** the present invention relates to a wound dressing comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows a change in the L value $\Delta$ L as defined in subsection 6.2.3 of ASTM D2244-16 of 10% or less, preferably 5% or less, further preferably 3% or less, over a time period of ten weeks, wherein the wound dressing is exposed to a temperature of 30°C in ambient air, at a humidity of 75%, over the entire ten week period, wherein the wound dressing has been sterilized prior to the 10 week period and wherein the $\Delta$ L value is measured as an average of at least ten measurements on 5 different spots on a 10 cm times 10 cm sample of the wound dressing.

**BRIEF DESCRIPTION OF THE DRAWINGS**

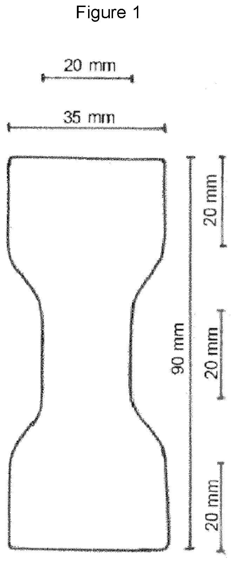

**Figure 1** is a schematic representation of a test piece used for determining the wet tensile strength.

**Figure 2** shows discoloration of a coated fiber in accordance with the present invention (sample "E") compared to a sample "A" (uppermost darker curve) of a fiber that is only coated with silver (and thus has a lower wet tensile strength as "E") and a sample "C" (lowermost curve)that is not obtained in accordance with the process of the present invention and shows significant discoloration,

**Figure 3** shows a wound dressing that is not in accordance with the present invention as the antimicrobial agent (silver) and the agent capable of forming a hydrogen bond are coated concomitantly onto the fibers (corresponding to sample "C" of Figure 2); as can be taken form Figure 3, significant occurrences of spots and speckles are visible to the eye.

## DETAILED DESCRIPTION OF THE INVENTION

As noted above, it is an object of the present invention to provide fiber materials (in particular fiber materials for wound therapy, in particular fiber materials comprised in a wound dressing) exhibiting, at the same time, improved mechanical and antimicrobial properties as well as improved wear properties (in the terms of "softness") without the occurrence of severe discolorations, preferably without any significant discoloration.

This object is particularly achieved by means of the inventive process, wherein a substrate is **first** contacted with an agent capable of forming a hydrogen bond and **then,** in a further step, which is performed separate from and **after** the first step, the obtained substrate is contacted with a silver-containing antimicrobial coating composition that is configured to avoid gelation of the substrate. The inventive process for making a modified fiber material is thus defined as follows:

(1) providing a substrate comprising or consisting of absorbent fibers;
(2) contacting the substrate with at least one agent having at least one, preferably at least two, groups capable of forming a hydrogen bond;
(3) contacting the product of step (2) with an antimicrobial coating composition comprising at least one silver-containing antimicrobial agents and one or more polymers in a solvent system that comprises a non-aqueous solvent.
(4) drying the product of step (2) or the product of step (3), or both.

The above object is further achieved by means of a fiber material, in particular a fiber material for use in a wound dressing, obtained or obtainable by the inventive process as described above and hereinunder.

The above object is further achieved by means of a wound dressing, comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows a change in the L value $\Delta$ L as defined in subsection 6.2.3 of ASTM D2244-16 of 10% or less, preferably 5% or less, further preferably 3% or less, over a time period of ten weeks, wherein the wound dressing is exposed to a temperature of 30°C in ambient air, at a humidity of 75%, over the entire ten week period, wherein the wound dressing has been sterilized prior to the 10 week period and wherein the $\Delta$ L value is measured as an average of at least ten measurements on 5 different spots on a 10 cm times 10 cm sample of the wound dressing.

The above object is further achieved by means of a wound dressing, comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows less than 0.5, preferably less than 0.1, further preferably no spots or speckles, as visible to the eye, in 10 different segments of an area of $1cm^2$, respectively, as present on a wound dressing of an overall area of $10cm^2$, wherein the number of spots or speckles as counted in all 10 segments is divided by 10 and wherein the spots or speckles as visible to the eye are determined after the lapse of a time period of ten weeks in which the wound dressing is exposed to a temperature of 30°C in ambient air, at a humidity of 75%, over the entire ten week period, wherein the wound dressing has been sterilized prior to the 10 week period.

As noted above, the inventors of the present application have surprisingly found that a silver-containing antimicrobial coating composition that provides for antimicrobial properties and overcomes the problem of substrate gelation in the presence of highly aqueous solvents (e.g. a coating composition known from WO 2017/016974) cannot simply be combined with an agent capable of forming a hydrogen bond (e.g. an agent described in WO 2018/137979, in particular glycerol), because that leads to a severe discoloration.

Without wishing to be bound by theory, it is believed that the severe discolorations resulting from combining the substrate, in a single step, with an antimicrobial coating composition that comprises all components in one mixture and in one solvent system that comprises a non-aqueous solvent (i.e. the at least one silver-containing antimicrobial agents, the one or more polymers, and the at least one agent having at least one, preferably at least two, groups capable of forming a hydrogen bond) are caused by interactions between the at least one agent having at least one, preferably at least two, groups capable of forming a hydrogen bond (e.g. glycerol) and other components of the compositions, in particular the silver-containing antimicrobial agent. In particular, it is assumed that the groups capable of forming a hydrogen bond (e.g. -OH groups or -COOH groups) interact with other components of the composition, in particular the silver-containing antimicrobial agent. That interaction, however, can be avoided if the agent having at least one, preferably at least two, groups capable of forming a hydrogen bond is contacted with the substrate **before** the other components (i.e. the antimicrobial coating composition comprising the silver-containing antimicrobial agent and the one or more polymers) are contacted with the substrate. It is assumed that in this way, the agent having at least one, preferably at least two, groups capable of forming a hydrogen bond first penetrates into the substrate and fibers thereof and forms hydrogen bonds with respective functional groups of the substrate such that the groups capable of forming a hydrogen bond are involved in hydrogen bonds and thus not available anymore for interacting with other components of the coating compositions, in particular not with the silver-containing antimicrobial agent. Thus, in the inventive process, interactions between the agent having at least one, preferably at least two, groups capable of forming a hydrogen bond and the components of the antimicrobial coating composition can be avoided or at least minimized.

In accordance with the present invention, and also in accordance with the universally accepted definition of the term, a *"hydrogen bond"* in the feature *"at least one group capable of forming a hydrogen bond"* is to be understood as relating to an attractive interaction between a hydrogen atom from a molecule or a molecular fragment X-H in which X is more electronegative than H, and an atom or a group of atoms in the same or a different molecule, in which there is evidence of bond formation (see IUPAC Definition for "hydrogen bond" in Pure Appl. Chem., Vol. 83, No. 8, pages 1637-1641, 2011).

As known to the skilled person, and as summarized in the above-mentioned IUPAC reference, a group capable of forming a hydrogen bond can be readily identified using one or any set of the following officially recognized experimental criteria (E1) - (E6):

- (E1) The forces involved in the formation of a hydrogen bond X- H ••• Y-Z include those of an electrostatic origin, those arising from charge transfer between the donor and acceptor leading to partial covalent bond formation between H and Y, and those originating from dispersion.
- (E2) The atoms X and H are covalently bonded to one another and the X-H bond is polarized, the H • • • Y bond strength increasing with the increase in electronegativity of X.
- (E3) The X - H • • • Y angle is usually linear (180°) and the closer the angle is to 180°, the stronger is the hydrogen bond and the shorter is the H • • • Y distance.
- (E4) The length of the X-H bond usually increases on hydrogen bond formation leading to a red shift in the infrared X-H stretching frequency and an increase in the infrared absorption cross-section for the X-H stretching vibration. The greater the lengthening of the X-H bond in X - H • • • Y, the stronger is the H • • • Y bond. Simultaneously, new vibrational modes associated with the formation of the H • • • Y bond are generated.
- (E5) The X- H • • • Y-Z hydrogen bond leads to characteristic NMR signatures that typically include pronounced proton deshielding for H in X-H, through hydrogen bond spin-spin couplings between X and Y, and nuclear Overhauser enhancements.
- (E6) The Gibbs energy of formation for the hydrogen bond should be greater than the thermal energy of the system for the hydrogen bond to be detected experimentally.

As noted above, one important feature of the present invention is that the substrate is **first** contacted with an agent capable of forming a hydrogen bond and **then,** in a further step performed **separate from** and **after** the first step, the thus obtained substrate is contacted with a silver-containing antimicrobial coating composition that is configured to avoid gelation of the substrate. Thus, processes wherein the agent capable of forming a hydrogen bond is applied to the substrate simultaneously with the silver-containing antimicrobial coating composition or processes wherein the silver-containing antimicrobial coating composition is applied to the substrate before the agent capable of forming a hydrogen bond is applied to the substrate are intended to be excluded from the present invention.

The present invention and the crucial steps of the inventive process are now described in detail:

## Step (2) of the inventive method

In step (2) of the inventive method, the substrate is contacted with an agent having at least one, preferably at least two, groups capable of forming a hydrogen bond.

In accordance with the present invention, the term *"contacting"* (or *"bringing into contact"*) is to be understood to generally relate to methods known to the person skilled in the art as relating to bringing a solid material into physical contact with another material, which may be a solid or a liquid. Examples of such 'contacting' include, but are not limited to: applying a solid or viscous substance by (spray) deposition, calendering, coating, dipping etc. or applying and/or spraying a fluid medium onto a (fiber) material, dipping the (fiber) material into a liquid medium, saturating/soaking of the (fiber) material in a liquid medium, or forming a slurry, suspension or mixture of the (fiber) material with a liquid medium. The liquid medium may for example be a mixture (e.g. suspension or solution) of the agent and at least one of the above discussed solvents.

In accordance with the present invention, it is particularly preferred that the substrate is impregnated with the at least one agent and/or that the at least one agent is applied onto the substrate.

According to a preferred embodiment, the agent having at least one, preferably at least two, groups capable of forming a hydrogen bond is provided as a compound that is dissolved or distributed in a liquid, in particular in form of a solution, a slurry, an emulsion or the like. In accordance with the present invention, it is particularly preferred that the agent is present as a solute in a solvent. The solvent or liquid in which the agent is dissolved/distributed is preferably a non-aqueous solvent/liquid so that gelation of the substrate is avoided in step (2). However, the direct application of the agent (compound), without a solvent, including in solid form, is also within the scope of the present invention.

Contacting the substrate with the agent having at least one, preferably at least two, groups capable of forming a hydrogen bond results in an improvement of wet tensile strength, which is believed due to the formation of hydrogen bonds between the agent and the fiber material (see WO 2018/137979).

In some embodiments, the product of step (4) in a wet state - i.e. the product of step (4) having absorbed a maximum amount of Solution A according to the "Free swell absorptive capacity method" (EN 13726-1; with an absorption time of $10\pm1$ minutes at $23\pm2$ °C) - has a wet tensile strength of at least 0.2 N/ 2cm.

The term *"wet tensile strength"* is to be understood as the maximum tensile force per unit width, as measured in accordance with EN 29073-3:1992 (with amendments as specified below), that a test piece will stand, in a wet state having absorbed a maximum amount of Solution A according to the "Free swell absorptive capacity method" EN 13726-1 (with an absorption time of $10\pm1$ minutes at $23\pm2$ °C), before it breaks apart in said tensile strength test.

For example, in some embodiments, the substrate in a wet state has a tensile strength of at least 0.4 N/ 2cm such as at least 0.6 N/ 2cm or at least 0.8 N/ 2cm or at least 1.0 N/ 2cm. In some embodiments, the substrate in a wet state has a tensile strength of at least 2 N/ 2cm, for example at least 2.5 N/ 2cm. In some embodiments, the substrate in a wet state has a tensile strength of at least 3 N/ 2cm, for example at least 3.5 N/ 2cm. In some embodiments, the substrate in a wet state has a tensile strength of at least 4 N/ 2cm, for example at least 4.5 N/ 2cm such as at least 5 N/ 2cm or at least 6 N/ 2cm or at least 7 N/ 2cm or at least 8 N/ 2cm or at least 9 N/ 2cm. In some embodiments, the substrate in a wet state has a tensile strength of at least 10 N/ 2cm, for example at least 15 N/ 2cm such as at least 20 N/ 2cm or at least 25 N/ 2cm. In some embodiments, the substrate in a wet state has a tensile strength of from 0.2 to 15 N/ 2cm. In some embodiments, the substrate in a wet state has a tensile strength of from 0.2 to 10 N/ 2cm. In some embodiments, the substrate in a wet state has a tensile strength of from 0.2 to 5 N/ 2cm. In some embodiments, the substrate in a wet state has a tensile strength of from 1 to 4 N/ 2cm.

Solution A, as defined in EN 13726-1, consists of a sodium chloride and calcium chloride solution containing 142 mmol of sodium ions and 2,5 mmol of calcium ions as the chloride salts. This solution has an ionic composition comparable to human serum or wound exudate. Said solution is prepared by dissolving 8.298 g of sodium chloride and 0.368 g of calcium chloride dihydrate in deionized water up to the "1 L" marking in a volumetric flask.

The method of measuring the "wet tensile strength", according to the invention, is modified vis-à-vis EN 29073-3:1992 in the following manner: i) the material is soaked according to EN 13726-1 (with an absorption time of $10\pm1$ minutes at $23\pm2$ °C and before cutting the test piece); ii) the test piece is cut according to Figure 1 giving a width of 20 mm; iii) a gauge length of 50 mm (i.e. distance between jaws according to section 7.2 of EN 29073-3:1992) is used; and iv) method is performed at a temperature of $23\pm2$ °C and $50\pm5$%rh. These deviations are all allowable alterations of EN 29073-3:1992 (see, e.g., notes in sections 6 and 7 of EN 29073-3:1992).

In some embodiments, the wet tensile strength (as defined above) is increased by at least 5%, preferably at least 10% and further preferably by at least 15%, for a fiber material according to the present invention, compared to an otherwise identical fiber material, which, however, has not been treated with the at least one agent comprising at least one group capable of forming a hydrogen bond, preferably at least two groups capable of forming a hydrogen bond and the antimicrobial coating composition.

*Step (2a)*

Step (2) of the inventive process may comprise a step (2a), which is performed after contacting the substrate with the agent capable of forming a hydrogen bond and before step (3). Step (2a) is a step of conditioning the product obtained by contacting the substrate with the agent capable of forming a hydrogen bond. In principle no restriction exist in regard to this conditioning other than that a certain time interval needs to pass, or the temperature needs to be increased above room temperature, or both. Exemplary and preferred temperature ranges and time intervals are provided below. The exact implementation on these conditions depends on the processing of the substrate, for example whether the substrate is present as a roll, for example a roll or a large diameter. In such cases, which are typically encountered in an actual manufacturing setting, conditioning times may be longer than under laboratory conditions. The temperature chosen will depend on the specific materials chosen. For example, the temperature should not be so high as to lead to significant evaporation of solvent or agent capable of forming hydrogen bonds. Also, the temperature should not be so high as to lead to a significant or even noticeable deterioration of materials, for example the substrate used. Hence, temperatures above 150°C are generally not suitable for conditioning/tempering.

Step (2a) may be realized as a step of tempering the product obtained from contacting the substrate with an agent having at least one, preferably at least two, groups capable of forming a hydrogen bond, at a temperature of at least 30 °C, or as a step of storing the product obtained from contacting the substrate with an agent having at least one, preferably at least two, groups capable of forming a hydrogen bond at a temperature of 18 °C to less than 30 °C.

Step (2a) has multiple effects. One effect is that, if the agent having at least one, preferably at least two, groups capable of forming a hydrogen bond is present in a (non-aqueous) solvent, the solvent is removed in step (2a) such that only the agent having at least one, preferably at least two, groups capable of forming a hydrogen bond remains in the substrate after step (2a), thereby preventing potential side reactions. A further effect is that interactions between the agent having at least one, preferably at least two, groups capable of forming a hydrogen bond and the substrate are formed and the number of such interactions are maximized such that the number of groups capable of forming a hydrogen bond that may interact with components of the antimicrobial coating composition is minimized, thereby leading to a further reduced discoloration.

The application of step (2a) is particularly preferred if the agent having at least one, preferably at least two, groups capable of forming a hydrogen bond is present in a solvent (preferably a non-aqueous solvent). However, it is within the scope of the present invention that said agent is used as such, i.e. without any solvent or only with trace amounts of solvent.

If step (2a) is a step of tempering the product obtained from contacting the substrate with an agent having at least one, preferably at least two, groups capable of forming a hydrogen bond, at a temperature of at least 30 °C, step (2a) is preferably performed at a temperature of 30 °C to 120 °C, preferably 40 °C to 110 °C, more preferably 50 °C to 100 °C, more preferably 60 °C to 90 °C, more preferably 70° C to 90 °C, more preferably 75 °C to 85 °C.

Preferably, tempering step (2a) is performed for a duration of 5 min to 90 min, preferably 10 min to 60 min, more preferably 20 min to 40 min, or for longer times of up to about 24 or up to about 48 hours ("min" stands for minutes). Tempering for longer periods of time may be advantageous on an industrial scale, for example in case large rolls of substrate are or need to be tempered.

A person of skill in the art understands that the duration of step (2a) may be adjusted depending on the nature of the non-aqueous solvent, if present, and the tempering temperature. Table 1 shows suitable combinations of temperatures and durations for the case that the agent having at least one, preferably at least two, groups capable of forming a hydrogen bond is present in an alcohol, in particular methanol, ethanol or propanol (including both n-propanol and isopropanol):

| Tempering temperature | Duration |
|---|---|
| 30 °C to 50 °C | 1 hour to 48 hours, preferably 2 hours min to 36 hours, more preferably 6 hours to 24 hours. |
| 50 °C to 60 °C | 20 min to 240 min, preferably 40 min to 180 min, more preferably, 60 min to 120 min. |
| 60 °C to 70 °C | 10 min to 120 min, preferably 10 min to 90 min |
| 70 °C to 90 °C | 10 min to 90 min, preferably 10 min to 60 min, more preferably 20 min to 40 min. |
| 75 °C to 85 °C | 5 min to 60 min, preferably 5 min to 40 min, more preferably 5 min to 20. |
| 90 °C to 110 °C | 1 min to 20 min, preferably 1 min to 10 min |

A person of skill in the art is able to identify suitable combinations of temperature and duration, i.e. a suitable combination of "tempering" and "storing".

In general, the tempering time at a certain temperature is not limiting. Thus, for example, a tempering temperature of 100 °C may also be applied for 24 hours without compromising the properties of the obtained product. However, it has been shown that there is a certain point in time after which no further improvements are achieved. Without being bound by theory, it is believed that at that point in time, the solvent is completely evaporated (if present) and all interactions between the agent having at least one, preferably at least two, groups capable of forming a hydrogen bond and the substrate have been formed such that a prolonged tempering does not lead to any further benefits. Thus, the above-mentioned combinations of temperatures and durations are to be understood to be those combinations that result in a desired product while minimizing economical disadvantages due to an unnecessary long tempering (e.g. storage costs, electricity costs, ...).

Preferably, tempering step (2a) is performed in a convection oven.

Tempering Step (2a) may be performed at reduced pressure (i.e. a pressure lower than the atmospheric pressure at otherwise identical conditions) so that the evaporation of solvent/liquid (if present)is accelerated compared to a situation where no reduced pressure is applied. Such a reduced pressure can be achieved by means of applying a vacuum, for example.

Preferably, tempering step (2a) is performed for a prolonged period of time. That functions for forming and maximizing the number of interactions between the agent having at least one, preferably at least two, groups capable of forming a hydrogen bond and the substrate. In this way, it is possible to minimize the number of groups capable of forming a hydrogen bond available for interacting with the components of the antimicrobial coating composition applied in step (3). Preferably, step (2a) is performed for period of 5 min to 90 min, preferably 10 min to 60 min, more preferably 20 min to 40 min, more preferably about 30 min.

It is understood that the temperature used in tempering step (2a) can be varied over time. That means that, for example, if the agent having at least one, preferably at least two, groups capable of forming a hydrogen bond is present in a solvent (preferably a non-aqueous solvent), a first temperature can be used for removing the solvent and then, a second temperature, which is different from the first temperature, can be used for further tempering the thus obtained product.

According to a preferred embodiment, a temperature gradient is applied in tempering step (2a). In this case, the temperature is preferably increased from 50 °C to 80 °C over time.

If step (2a) is a step of storing the product obtained from contacting the substrate with an agent having at least one, preferably at least two, groups capable of forming a hydrogen bond, at a temperature of 18 °C to less than 30 °C, the product is preferably stored at a temperature of preferably 20 °C to less than 30 °C, more preferably at about 20 °C ("room temperature").

Due to the lower temperature used in the storing step (2a) compared to the tempering step (2a), storing step (2a) is generally applied for a longer period of time compared to tempering step (2a). Preferably, the step of storing the product obtained from contacting the substrate with an agent having at least one, preferably at least two, groups capable of forming a hydrogen bond at a temperature of 18 °C to less than 30 °C, preferably 20 °C to less than 30 °C, more preferably about 20 °C (room temperature), is performed for a duration of 1 week to 40 weeks, preferably 4 weeks to 30 weeks.

Storing step (2a) may also be performed at reduced pressure (i.e. a pressure lower than the atmospheric pressure at otherwise identical conditions) so that the evaporation of solvent/liquid (if present) is accelerated compared to a situation where no reduced pressure is applied. Such a reduced pressure can be achieved by means of applying a vacuum, for example.

Also storing step (2a) is performed for a prolonged period of time. As described above for tempering step (2a), that functions for forming and maximizing the number of interactions between the agent having at least one, preferably at least two, groups capable of forming a hydrogen bond and the substrate. In this way, it is possible to minimize the number of groups capable of forming a hydrogen bond available for interacting with the components of the antimicrobial coating composition applied in step (3).

Preferably, step (3) is performed more than 10 minutes, preferably more than 15 minutes, more preferably more than 20 minutes, more preferably more than 30 minutes, more preferably more than 40 minutes, more preferably more than 50 minutes, more preferably more than 60 minutes, after the substrate has been contacted with the agent having at least one, preferably at least two, groups capable of forming a hydrogen bond, i.e. after conclusion of step (2)

That means that if tempering step (2a) is present, step (2a) is preferably performed for a period of more than 10 minutes, preferably more than 15 minutes, more preferably more than 20 minutes, more preferably more than 30 minutes, more preferably more than 40 minutes, more preferably more than 50 minutes, more preferably more than 60 minutes.

If, however, the storing step (2a) is performed instead of the tempering step (2a), the product obtained in step (2) is preferably stored at room temperature (a vacuum may be applied) and further preferably for a period of at least one week, preferably at least 10 weeks; or wherein said step of storing is performed from 1 week to 40 weeks, preferably from 4 weeks to 30 weeks.

### Step (3) of the inventive method

In step (3) of the inventive method, the product of step (2) is contacted with an antimicrobial coating composition comprising at least one silver-containing antimicrobial agent and one or more polymers in a solvent system that comprises a non-aqueous solvent.

As noted above, the silver-containing antimicrobial coating composition renders the fiber material antimicrobially active while at the same time overcoming the problem that silver-based antimicrobial agents are usually only soluble in highly aqueous solutions while polymers useful for the production of absorbent wound dressings are incompatible with highly aqueous solutions because they gel in the presence of water. That problem is solved by using an antimicrobial coating composition that comprises (i) a non-aqueous solvent that avoids or reduces the amount of absorption that takes place during application of the antimicrobial agent and (ii) one or more polymers that help disperse the antimicrobial agent and avoid settling thereof. In addition to that, the one or more polymers in the antimicrobial coating on the wound dressing substrate modulate the rate at which the antimicrobial agent is released from the resulting wound dressing.

The antimicrobial coating composition can be prepared as described in WO 2017/016974, the respective content of which is herewith incorporated by reference. Contacting step (3) is preferably performed by using slot die, foulard, or kiss coating. In some embodiments, contacting step (3) is performed using slot die coating. In some embodiments, contacting step (b) is performed using foulard coating. In some embodiments, contacting step (3) is performed using kiss coating.

### *Step (4) of the inventive method*

Step (4) of the inventive method is a step of drying the product of step (2) or of step (3), or both.

In some embodiments, drying step (4) is performed by subjecting the product of step (2) or of step (3), or both to a hot air convection oven or hot plates. In some embodiments, drying step (4) is performed by passing the product of step (3) through a hot air convection oven. In some embodiments, drying step (4) is performed by passing the product of step (3) over hot plates.

Suitable substrates, agents capable of forming a hydrogen bond, and antimicrobial coating compositions that may be used in the present invention are described below.

### *Substrate*

In accordance with the present invention, the substrate is a structure that has a larger extension in a (x-y) plane than in the direction perpendicular thereto (z-direction). Preferably the substrate has an area weight ("grammage") of 20 - 800 $g/m^2$, preferably 100 - 450 $g/m^2$.

It is preferred that the substrate is a substrate of a wound dressing.

The substrate comprises or consists of absorbent fibers.

In accordance with the present invention, the term *"fiber"* is to be understood as generally referring to threads or threadlike structures and is generally understood to relate to a flexible structure, which is thin in relation to its length. Fibers have a small diameter and can be built up with one another by corresponding bonding processes to produce fibrous structures or fiber materials. In accordance with the present invention, the average diameter of the fibers making up the fiber material is preferably in the range of 50 nm to 1000 $\mu$m, preferably 1 $\mu$m to 100 $\mu$m, further preferably from 5 - 35 $\mu$m.

Preferably, the substrate is a non-woven substrate. In accordance with the present invention, and also in accordance with the universally accepted understanding of the skilled person, a "non-woven substrate" is defined as sheet or web structures bonded together by entangling fiber or filaments mechanically, thermally, or chemically, but not (as is conventionally done for fabrics) by weaving or knitting. Nonwovens are defined by ISO standard 9092 and CEN EN 29092. Non-woven sheets or webs are typically flat, porous sheets that are made directly from separate fibers or from molten plastic or plastic films.

The substrate may further comprise absorbent particles, such as superabsorbent particles. As used herein, the term "superabsorbent particles" denotes particles which can absorb at least 10 times their own weight in distilled water. In some embodiments, the superabsorbent particles comprise poly-N-vinylpyrrolidone, polyvinyltoluene sulfonate, polysulfoethylacrylate, poly-2-hydroxyethyl acrylate, polyvinylmethyloxazolidinone, polyacrylamide, polyacrylic acid, polymethacrylic acid, or copolymers or terpolymers of polysaccharides, polyacrylic acid, polyacrylamide, or polymethacrylic acid. In some embodiments, the superabsorbent particles comprise polyacrylic acid. The presence of absorbing particles allows to further control fluid uptake and management capabilities of the fiber material, which is particularly advantageous in wound treatment.

In some embodiments, the substrate also includes non-absorbent fibers. In some embodiments, the absorbent fibers and/or absorbent particles are airlaid by spraying, needling, or carding together with non-absorbent fibers

In some embodiments of the invention, the substrate has a free swell absorptive capacity, corresponding to the maximum absorptive capacity of the substrate, of at least 1 times its own weight as measured by EN 13726-1:2002 ("Free swell absorptive capacity"). For example, in some embodiments, the substrate has a free swell absorptive capacity, corresponding to the maximum absorptive capacity of the substrate, of at least 3 times its own weight as measured by EN 13726-1:2002. For example, in some embodiments, the substrate has a free swell absorptive capacity, corresponding to the maximum absorptive capacity of the substrate, of at least 5 times its own weight as measured by EN 13726-1:2002. For example, in some embodiments, the substrate has a free swell absorptive capacity, corresponding to the maximum absorptive capacity of the substrate, of at least 10 times its own weight as measured by EN 13726-1:2002.

Preferably, the fibers comprise or consist of (preferably consist of) a polymer. Preferably, the polymer is crosslinked.

The term *"crosslinked"* is used herein to describe a material comprising a plurality polymer molecules which are interlinked by a chemical bond, in particular a covalent bond or an ionic bond, or by a physical cross-link, such as in thermoplastic elastomers.

In some embodiments, the material is cross-linked by heat or chemical treatment, in particular by heat. Cross-linked fiber materials are capable of forming a swollen coherent gel upon absorbing a liquid. Thereby, a wound dressing substrate comprising such a fiber material can be removed coherently from a wound. Using fiber material that is (already) crosslinked is in accordance with the present invention, in regard to which it is believed that the bringing into contact of the non-ionic fiber material with the at least one agent comprising one, preferably two groups capable of forming a hydrogen bond, results in (further) crosslinking of the fiber material.

The substrate preferably comprises or consists of polyvinyl alcohol, a polysaccharide, polyacrylic acid, polymethacrylic acid, and a copolymer comprising two or more monomers selected from vinyl alcohol, acrylic acid, and methacrylic acid. Preferably, the substrate comprises or consists of polyvinyl alcohol. For example, in some embodiments the absorbent fibers may comprise or consist of a plurality of fibers comprising polyvinyl alcohol, such as the plurality of fibers disclosed in US 2013/0323195 and/or US 2013/0274415, hereby incorporated by reference. It is particularly preferred that the substrate is a web of non-woven polyvinyl alcohol fibers, wherein the polyvinyl alcohol is preferably crosslinked.

A polyvinyl alcohol copolymer may be used in unsubstituted or in partially substituted form. In the event of partial substitution, partial substitution of the OH groups by -O(C=O)-R or -OR is included, wherein R, in each case independently of one another, stands for a C1-C4 alkyl group. In this case a C1-C4 alkyl group is understood to be methyl, ethyl, propyl, iso-propyl, 1-butyl, 2-butyl, or tert-butyl.

Polyethylene vinyl alcohol, polyvinyl alcohol styrene, polyvinyl alcohol vinyl acetate, polyvinyl alcohol vinyl pyrrolidone, polyvinyl alcohol ethylene glycol and/or polyvinyl alcohol, particularly preferably polyethylene vinyl alcohol, polyvinyl alcohol vinyl acetate, polyvinyl alcohol vinyl pyrrolidone, polyvinyl alcohol vinylamine, polyvinyl alcohol acrylate, polyvinyl alcohol acrylamide, polyvinyl alcohol ethylene glycol and/or polyvinyl alcohol and may also be used as fiber material, in accordance with the present invention. Block copolymers and/or graft copolymers and/or block and graft copolymers, statistical or alternating systems and any mixtures of these may also be used as non-ionic fiber material.

In some embodiments, the absorbent fibers and/or absorbent particles (if present) comprise or consist of polyacrylic acid. In some embodiments, the absorbent fibers and/or absorbent particles (if present) comprise or consist of polymethacrylic acid. In some embodiments, the absorbent fibers and/or absorbent particles (if present) comprise or consist of a copolymer comprising two or more monomers selected from vinyl alcohol, acrylic acid, and methacrylic acid. In some embodiments, the absorbent fibers and/or absorbent particles (if present) comprise or consist of polysaccharides. In some embodiments, the polysaccharides are selected from the group consisting of cellulosic polymers, alginates, alginic acid, amylopectins, amyloses, beta-glucans, carrageenan, chitosans, gellan gums, gelatins, pectic acid, pectin, and xanthan gum. In some embodiments, the absorbent fibers and/or absorbent particles (if present) comprise a cellulosic polymer. In some embodiments, the absorbent fibers and/or absorbent particles (if present) comprise or consist of carboxymethyl cellulose.

In some embodiments, the absorbent fibers and/or absorbent particles (if present) comprise a polymer that is cross-linked. In some embodiments, the absorbent fibers and/or absorbent particles (if present) comprise or consist of cross-linked polyvinyl alcohol. In some embodiments, the absorbent fibers and/or absorbent particles (if present) are cross-linked by heat or chemical treatment. In some embodiments, the absorbent fibers and/or absorbent particles (if present) are cross-linked by heat. In some embodiment the wound dressing substrate may comprise cross-linked absorbent fibers, wherein the cross-linked absorbent fibers are capable of forming a swollen coherent gel upon absorbing a liquid. Thereby, the wound dressing substrate can be removed coherently from a wound.

Furthermore, the substrate may be formed as a polymer blend. In this case a polymer blend is understood to be a physical mixture of at least two polymers from the melt or from the solution.

In order to form such a polymer blend, *further polymers* may be used, such as for example alginates, cellulose ethers, such as carboxymethyl celluloses, methyl celluloses, ethyl celluloses, hydroxymethyl celluloses, hydroxyethyl celluloses, hydroxyalkyl methylcelluloses, hydroxypropyl celluloses, cellulose esters, such as cellulose acetate, oxidised celluloses, bacterial celluloses, cellulose carbonates, gelatines, collagens, starches, hyaluronic acids, pectins, agar, polyacrylates, polyvinyl amines, polyvinyl acetates, polyethylene glycols, polyethylene oxides, polyvinyl pyrrolidones, polyurethanes or nongelling further polymers, such as for example polyolefins, celluloses, cellulose derivatives, regenerated celluloses such viscoses, polyamides, polyacrylonitriles, polyvinyl chlorides, chitosans, polylactides, polyglycolides, polyester amides, polycaprolactones, polyhexamethylene terephthalates, polyhydroxybutyrates, polyhydroxyvalerates or polyesters.

*Agent having at least one, preferably at least two, groups capable of forming* a *hydrogen bond*

As noted above, the agent having at least one, preferably at least two, groups capable of forming a hydrogen bond maybe provided "as such", i.e. without any (or only trace amounts) of solvents or adjuvants, but is preferably provided as a compound that is dissolved or distributed in a liquid, in particular in form of a solution, a slurry, an emulsion or the like. In accordance with the present invention, it is particularly preferred if the agent is present as a solute in a solvent. The solvent or liquid is preferably a non-aqueous solvent/liquid such that a gelation of the substrate is avoided in step (2). Examples of such solvents/liquids are, but are not limited to, ethanol, isopropanol and methanol. Ethanol is a preferred solvent/liquid.

Preferably, the concentration of the agent in the solvent/liquid is 10 to 100 wt.%, more preferably 20 to 80 wt.%, more preferably 30 to 70 wt.%, more preferably 40 to 60 wt.%, more preferably 45 to 55 wt.%. The concentration is calculated according to the following formula:

$$[c] = [(\text{weight of agent}) / (\text{weight of agent} + \text{weight of solvent/liquid})] \times 100,$$

wherein
[c] is the concentration of the agent in wt.%

In principle, no limitations exist in regard to the agent that comprises at least one group capable of forming a hydrogen bond, preferably at least two groups capable of forming a hydrogen bond. Said agent is preferably a compound, which compound can either be a monomer / small molecule or a polymer.

In some embodiments, the agent that comprises at least one group capable of forming a hydrogen bond, preferably at least two groups capable of forming a hydrogen bond, is selected from the group consisting of polyols, in particular sugar alcohols (sugar polyols), polymeric polyols; polysaccharides, alpha-hydroxy acids; cellulose ethers or cellulose esters; di- or polyisocyanates; polyethers or polyesters.

Non-limiting examples of such agents are: glycol, propylene glycol, glycerol, sorbitol, xylitol, maltitol, hexylene glycol, butylene glycol, glyceryl triacetate, polydextrose, lactic acid, panthtothenic acid, hyaluronic acid, sodium-2-pyrrolidone-5- carboxylate ('sodium PCA'), polyethylene glycol (PEG) (also known as polyethylene oxide or polyoxyethylene), polypropylene glycol (PPG), polyvinylpyrrolidone (PVP), (also known as polyvidone or povidone).

In some embodiments, the agent that comprises at least one group capable of forming a hydrogen bond, preferably at least two groups capable of forming a hydrogen bond, is glycerol or polyethylene glycol, preferably glycerol.

*Antimicrobial coating composition*

In general, the antimicrobial coating composition comprises one or more silver-containing antimicrobial agents and one or more polymers in a solvent system that comprises a non-aqueous solvent.

The term *"antimicrobial coating composition"* as used herein is different from and must be distinguished from the term *"antimicrobial coating"* in that "antimicrobial coating" refers to the mixture of antimicrobial agent and one or more polymers that have been applied to the substrate and dried. Thus, the antimicrobial coating can be thought of as the residue that remains on the substrate after the antimicrobial coating composition is applied to the substrate and the substrate is dried to remove the solvents.

The antimicrobial coating composition can be prepared as described in WO 2017/016974, the respective content of which is herewith incorporated by reference.

*The silver-containing antimicrobial agent*

The antimicrobial agent comprises silver.

In some embodiments, the silver is present in the form of metallic silver. In some embodiments, the silver is present in the form of a silver salt.

In some embodiments, the silver salt is silver sulfate, silver chloride, silver nitrate, silver sulfadiazine, silver carbonate, silver phosphate, silver lactate, silver bromide, silver acetate, silver citrate, silver CMC, silver oxide. Preferably, the antimicrobial agent is silver sulfate.

The antimicrobial coating composition may comprise further antimicrobial agents in addition to the silver-containing antimicrobial agent. For example, the antimicrobial coating composition may comprise iodine. In some embodiments, the iodine is povidone iodine, cadexomer iodine, triocyn, or iodozyme.

In some embodiments, the antimicrobial coating composition may comprise a monoguanide or biguanide. In some embodiments the monoguanide or biguanide is chlorhexidine digluconate, chlorhexidine diacetate, chlorhexidine dihydrochloride, polyhexamethylenebiguanide (PHMB) or a salt thereof, or polyhexamethylenemonoguanide (PHMG) or a salt thereof. In some embodiments the biguanide is PHMB or a salt thereof.

In some embodiments, the antimicrobial coating composition may comprise a quaternary ammonium compound. In some embodiments, the quaternary ammonium compound is cetylpyridinium chloride, benzethonium chloride, or poly-DADMAC.

In some embodiments, the antimicrobial coating composition may comprise triclosan, sodium hypochlorite, copper, hydrogen peroxide, xylitol, or honey.

In some embodiments, the silver-containing antimicrobial agent in the antimicrobial coating composition is present in an amount less than 40% w/w. In some embodiments, the silver-containing antimicrobial agent in the antimicrobial coating composition is present in an amount less than 35% w/w. In some embodiments, the silver-containing antimicrobial agent in the antimicrobial coating composition is present in an amount less than 30% w/w. In some embodiments, the silver-containing antimicrobial agent in the antimicrobial coating composition is present in an amount less than 25% w/w. In some embodiments, the silver-containing antimicrobial agent in the antimicrobial coating composition is present in an amount less than 20% w/w. In some embodiments, the silver-containing antimicrobial agent in the antimicrobial coating composition is present in an amount less than 15% w/w. In some embodiments, the silver-containing antimicrobial agent in the antimicrobial coating composition is present in an amount less than 10% w/w. In some embodiments, the silver-containing antimicrobial agent in the antimicrobial coating composition is present in an amount less than 5% w/w.

In some embodiments, the silver-containing antimicrobial agent in the antimicrobial coating composition is present in an amount from 0.1 %t to 40% w/w. In some embodiments, the silver-containing antimicrobial agent in the antimicrobial coating composition is present in an amount from 0.1% to 35%. In some embodiments, the silver-containing antimicrobial agent in the antimicrobial coating composition is present in an amount from 0.1% to 30%. In some embodiments, the silver-containing antimicrobial agent in the antimicrobial coating composition is present in an amount from 0.1% to 25%. In some embodiments, the silver-containing antimicrobial agent in the antimicrobial coating composition is present in an amount from 0.1% to 20%. In some embodiments, the silver-containing antimicrobial agent in the antimicrobial coating composition is present in an amount from 0.1% to 15%. In some embodiments, the silver-containing antimicrobial agent in the antimicrobial coating composition is present in an amount from 0.1% to 10%. In some embodiments, the silver-containing antimicrobial agent in the antimicrobial coating composition is present in an amount from 0.1% to 5%. In some embodiments, the silver-containing antimicrobial agent in the antimicrobial coating composition is present in an amount from 0.1% to 1 %. In some embodiments, the silver-containing antimicrobial agent in the antimicrobial coating composition is present in an amount from 0.5% to 3%. In some embodiments, the silver-containing antimicrobial agent in the antimicrobial coating composition is present in an amount from 1 % to 40%. In some embodiments, the silver-containing antimicrobial agent in the antimicrobial coating composition is present in an amount from 5% to 40%. In some embodiments, the silver-containing antimicrobial agent in the antimicrobial coating composition is present in an amount from 10% to 40%. In some embodiments, the silver-containing antimicrobial agent in the antimicrobial coating composition is present in an amount from 15% to 40%. In some embodiments, the silver-containing antimicrobial agent in the antimicrobial coating composition is present in an amount from 20% to 40%. In some embodiments, the silver-containing antimicrobial agent in the antimicrobial coating composition is present in an amount from 25% to 40%. In some embodiments, the silver-containing antimicrobial agent in the antimicrobial coating composition is present in an amount from 30% to 40%. In some embodiments, the silver-containing antimicrobial agent in the antimicrobial coating composition is present in an amount from 35% to 40%.

In some embodiments, the silver-containing antimicrobial agent in the substrate is present in an amount less than 30 mg/cm$^2$. In some embodiments, the silver-containing antimicrobial agent in the substrate is present in an amount less than 25 mg/cm$^2$. In some embodiments, the silver-containing antimicrobial agent in the substrate is present in an amount less than 20 mg/cm$^2$. In some embodiments, the silver-containing antimicrobial agent in the substrate is present in an amount less than 15 mg/cm$^2$. In some embodiments, the silver-containing antimicrobial agent in the substrate is present in an amount less than 10 mg/cm$^2$. In some embodiments, the silver-containing antimicrobial agent in the substrate is present in an amount less than 5 mg/cm$^2$. In some embodiments, the silver-containing antimicrobial agent in the substrate is present in an amount less than 1 mg/cm$^2$. In some embodiments, the silver-containing antimicrobial agent in the substrate is present in an amount less than 0.5 mg/cm$^2$.

In some embodiments, the silver-containing antimicrobial agent in the substrate is present in an amount from 0.01 mg/cm$^2$ to 30 mg/cm$^2$. In some embodiments, the silver-containing antimicrobial agent in the substrate is present in an amount from 0.01 mg/cm$^2$ to 35 mg/cm$^2$. In some embodiments, the silver-containing antimicrobial agent in the substrate is present in an amount from 0.01 mg/cm$^2$ to 30 mg/cm$^2$. In some embodiments, the silver-containing antimicrobial agent in the substrate is present in an amount from 0.01 mg/cm$^2$ to 25 mg/cm$^2$. In some embodiments, the silver-containing antimicrobial agent in the substrate is present in an amount from 0.01 mg/cm$^2$ to 20 mg/cm$^2$. In some embodiments, the silver-containing antimicrobial agent in the substrate is present in an amount from 0.01 mg/cm$^2$ to 15 mg/cm$^2$. In some embodiments, the silver-containing antimicrobial agent in the substrate is present in an amount from 0.01 mg/cm$^2$ to 10 mg/cm$^2$. In some embodiments, the silver-containing antimicrobial agent in the substrate is present in an amount from 0.01 mg/cm$^2$ to 5 mg/cm$^2$. In some embodiments, the silver-containing antimicrobial agent in the substrate is present in an amount from 0.05 mg/cm$^2$ to 3 mg/cm$^2$. In some embodiments, the silver-containing antimicrobial agent in the substrate is present in an amount from 0.05 mg/cm$^2$ to 1 mg/cm$^2$. In some embodiments, the silver-containing antimicrobial agent in the substrate is present in an amount from 0.1 mg/cm$^2$ to 1 mg/cm$^2$, for example, from 0.1 mg/cm$^2$ to 0.5 mg/cm$^2$. In some embodiments, the silver-containing antimicrobial agent in the substrate is present in an amount from 0.1 mg/cm$^2$ to 40 mg/cm$^2$. In some embodiments, the silver-containing antimicrobial agent in the substrate is present in an amount from 0.5 mg/cm$^2$ to 40 mg/cm$^2$. In some embodiments, the silver-containing antimicrobial agent in the substrate is present in an amount from 1 mg/cm$^2$ to 40 mg/cm$^2$. In some embodiments, the silver-containing antimicrobial agent in the substrate is present in an amount from 5 mg/cm$^2$ to 40 mg/cm$^2$. In some embodiments, the silver-containing antimicrobial agent in the substrate is present in an amount from 10 mg/cm$^2$ to 40 mg/cm$^2$. In some embodiments, the silver-containing antimicrobial agent in the substrate is present in an amount from 15 mg/cm$^2$ to 40 mg/cm$^2$. In some embodiments, the silver-containing antimicrobial agent in the substrate is present in an amount from 20 mg/cm$^2$ to 40 mg/cm$^2$. In some embodiments, the silver-containing antimicrobial agent in the substrate is present in an amount from 25 mg/cm$^2$ to 40 mg/cm$^2$. In some embodiments, the silver-containing antimicrobial agent in the substrate is present in an amount from 30 mg/cm$^2$ to 40 mg/cm$^2$. In some embodiments, the silver-containing antimicrobial agent in the substrate is present in an amount from 35 mg/cm$^2$ to 40 mg/cm$^2$.

*Polymer (in the coating composition)*

In some embodiments, the one or more polymers in the antimicrobial coating composition are selected from the group consisting of cellulosic polymers, neutral poly(meth)acrylate esters, polyvinylpyrrolidone, polyvinylpolypyrrolidone, and combinations thereof.

In some embodiments, the one or more polymers in the antimicrobial coating composition are selected from cellulosic polymers. In some embodiments, the one or more polymers in the coating composition are cellulosic polymers selected from hydroxypropylmethylcellulose (HPMC), hydroxypropylcellulose (HPC), methylcellulose (MC), ethylcellulose (EC), and combinations thereof. In some embodiments, one of the one or more polymers in the coating composition is hydroxypropylcellulose. In some embodiments, two of the polymers in the coating composition are hydroxypropylcellulose and ethylcellulose.

In some embodiments, at least one of the one or more polymers in the antimicrobial coating composition is a neutral poly(meth)acrylate ester. In some embodiments, at least one of the one or more polymers in the coating composition is a methyl methacrylate / ethyl acrylate copolymers (e.g., a EUDRAGIT polymer).

Preferably, the one or more polymer is HPC.

In some embodiments, at least one of the one or more polymers in the antimicrobial coating composition is water soluble. As used herein, a "water soluble polymer" is soluble in water at 25 degrees Celsius at a concentration of at least 1 grams per liter. As used herein, "a non-water soluble polymer" is soluble in water at 25 °C at a concentration of no more than 1 grams per liter. In some embodiments, the one or more polymers in the antimicrobial coating composition comprise a mixture of at least one water soluble polymer and at least one non-water soluble polymer.

In some embodiments, each of the one or more polymers in the antimicrobial coating composition is present in an amount less than 30% w/w. In some embodiments, each of the one or more polymers in the antimicrobial coating composition is present in an amount less than 20% w/w. In some embodiments, each of the one or more polymers in the antimicrobial coating composition is present in an amount less than 10% w/w. In some embodiments, each of the one or more polymers in the antimicrobial coating composition is present in an amount less than 5% w/w. In some embodiments, each of the one or more polymers in the antimicrobial coating composition is present in an amount less than 4% w/w. In some embodiments, each of the one or more polymers in the antimicrobial coating composition is present in an amount less than 3% w/w. In some embodiments, each of the one or more polymers in the antimicrobial coating composition is present in an amount less than 2% w/w. In some embodiments, each of the one or more polymers in the antimicrobial coating composition is present in an amount less than 1% w/w.

In some embodiments, each of the one or more polymers in the antimicrobial coating composition is present in an amount from 0.5% to 30% w/w. In some embodiments, each of the one or more polymers in the antimicrobial coating composition is present in an amount from 0.5% to 20% w/w. In some embodiments, each of the one or more polymers in the antimicrobial coating composition is present in an amount from 0.5% to 10% w/w. In some embodiments, each of the one or more polymers in the antimicrobial coating composition is present in an amount from 0.5% to 5% w/w. In some embodiments, each of the one or more polymers in the antimicrobial coating composition is present in an amount from 0.5% to 4% w/w. In some embodiments, each of the one or more polymers in the antimicrobial coating composition is present in an amount from 0.5% to 3% w/w. In some embodiments, each of the one or more polymers in the antimicrobial coating composition is present in an amount from 1% to 2% w/w.

In some embodiments, the one or more polymers in the antimicrobial coating composition have an average molecular weight from 50 - 1,500 kDa. In some embodiments, the one or more polymers in the antimicrobial coating composition have an average molecular weight from 300 - 1,500 kDa. In some embodiments, the one or more polymers in the antimicrobial coating composition have an average molecular weight from 500 - 1,500 kDa. In some embodiments, the one or more polymers in the antimicrobial coating composition have an average molecular weight from 800 - 1,500 kDa. In some embodiments, the one or more polymers in the antimicrobial coating composition have an average molecular weight from 800 - 900 kDa. In some embodiments, the one or more polymers in the antimicrobial coating composition have an average molecular weight from 800 - 1,000 kDa. In some embodiments, the one or more polymers in the antimicrobial coating composition have an average molecular weight from 800 - 1,200 kDa. In some embodiments, the one or more polymers in the antimicrobial coating composition have an average molecular weight from 1,000 - 1,200 kDa. In some embodiments, the one or more polymers in the antimicrobial coating composition have an average molecular weight from 1,100 - 1,200 kDa.

*Non-aaueous solvent*

In some embodiments, the non-aqueous solvent comprises a polar protic solvent. In some embodiments, the polar protic solvent comprises an alcohol. In some embodiments, the polar protic solvent comprises a $C_{1-4}$ alkyl alcohol. In some embodiments, the polar protic solvent comprises methanol, ethanol, *n*-propanol, isopropanol, *n*-butanol, or s-butanol. In some embodiments, the polar protic solvent comprises ethanol.

In some embodiments, the antimicrobial coating composition comprises water. In some embodiments, the antimicrobial coating composition comprises less than 50% w/w of water. In some embodiments, the antimicrobial coating composition comprises less than 40% w/w of water. In some embodiments, the antimicrobial coating composition comprises less than 30% w/w of water. In some embodiments, the antimicrobial coating composition comprises less than 20% w/w of water. In some embodiments, the antimicrobial coating composition comprises less than 15% w/w of water. In some embodiments, the antimicrobial coating composition comprises less than 10% w/w of water. In some embodiments, the antimicrobial coating composition comprises less than 5% w/w of water. In some embodiments, the antimicrobial coating composition comprises less than 1% w/w water. In some embodiments, the coating composition comprises only trace amounts of water.

In some embodiments, the antimicrobial coating composition comprises from 1% to 50% w/w of water. In some embodiments, the antimicrobial coating composition comprises from 1% to 40% w/w of water. In some embodiments, the antimicrobial coating composition comprises from 1% to 30% w/w of water. In some embodiments, the antimicrobial coating composition comprises from 1% to 20% w/w of water. In some embodiments, the antimicrobial coating composition comprises from 1% to 10% w/w of water. In some embodiments, the antimicrobial coating composition comprises from 10% to 50% w/w of water. In some embodiments, the antimicrobial coating composition comprises from 10% to 40% w/w of water. In some embodiments, the antimicrobial coating composition comprises from 10% to 30% w/w of water. In some embodiments, the antimicrobial coating composition comprises from 10% to 20% w/w of water. In some embodiments, the antimicrobial coating composition comprises from 20% to 50% w/w of water. In some embodiments, the antimicrobial coating composition comprises from 20% to 40% w/w of water. In some embodiments, the antimicrobial coating composition comprises from 20% to 30% w/w of water. In some embodiments, the antimicrobial coating composition comprises from 30% to 50% w/w of water. In some embodiments, the antimicrobial coating composition comprises from 30% to 40% w/w of water. In some embodiments, the antimicrobial coating composition comprises from 40% to 50% w/w of water.

In a second aspect, the present invention relates to a fiber material, in particular a fiber material comprised in a wound dressing, obtained by the inventive process. That fiber material is characterized in that it exhibits, at the same time, improved mechanical and antimicrobial properties as well as improved wear properties without showing any severe discoloration.

In a ***third aspect***, the present invention relates to a wound dressing, comprising a substrate comprising or consisting of absorbent fibers, at least one antimicrobial agent, at least one polymer, and an agent having at least one, preferably at least two, groups capable of forming a hydrogen bond. The fiber material is particularly characterized in that it does not show significant, let alone severe discoloration.

In particular, the third aspect relates to a wound dressing, comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows a change in the E (L,a,b) value of 5% or less, preferably 3% or less, over a time period of ten days, wherein the wound dressing is exposed to a temperature of 30°C in ambient air, at a humidity of 75%, over the entire ten day period, wherein the wound dressing has been sterilized prior to the 10 day period and wherein the E value is measured as an average of at least ten measurements on 10 different spots on a 10 cm times 10 cm sample of the wound dressing.

The third aspect also relates to a wound dressing comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows a change in the L value Δ L as defined in subsection 6.2.3 of ASTM D2244-16 of 10% or less, preferably 5% or less, further preferably 3% or less, over a time period of ten weeks, wherein the wound dressing is exposed to a temperature of 30°C in ambient air, at a humidity of 75%, over the entire ten week period, wherein the wound dressing has been sterilized prior to the 10 week period and wherein the Δ L value is measured as an average of at least five measurements on 5 different spots on a 10 cm times 10 cm sample of the wound dressing.

The third aspect also relates to a wound dressing comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows less than 0.5, preferably less than 0.1, further preferably no spots or speckles, as visible to the eye, in 10 different segments of an area of 1cm$^2$, respectively, as present on a wound dressing of an overall area of 10cm$^2$, wherein the number of spots or speckles as counted in all 10 segments is divided by 10 and wherein the spots or speckles as visible to the eye are determined after the lapse of a time period of ten weeks in which the wound dressing is exposed to a temperature of 30°C in ambient air, at a humidity of 75%, over the entire ten week period, wherein the wound dressing has been sterilized prior to the 10 week period.

The substrate, antimicrobial agent, polymer, and the agent having at least one, preferably at least two, groups capable of forming a hydrogen bond are as defined above.

The present invention is now further described by means of embodiments 1 to 57, which embodiments may be combined with any other embodiment or instance of disclosure in the description:

1. A process for making a modified fiber material, wherein the process comprises the following steps:

   (1) providing a substrate comprising or consisting of absorbent fibers;
   (2) contacting the substrate with an agent having at least one, preferably at least two, groups capable of forming a hydrogen bond;
   (3) contacting the product of step (2) with an antimicrobial coating composition comprising at least one silver-containing antimicrobial agent and one or more polymers in a solvent system that comprises a non-aqueous solvent.
   (4) drying the product of step (2) or the product of step (3), or both.

2. The process of embodiment 1, wherein the modified fiber material is part of a wound dressing.

3. The process of embodiment 1 or 2, wherein the product of step (4) has a wet tensile strength of at least 0.2 N/2cm, wherein said wet tensile strength is determined as set out in the description.

4. The process according to any one of embodiments 1-3, wherein the wet tensile strength of the product of step (4) is increased by at least 5%, preferably at least 10% and further preferably by at least 15%, compared to an otherwise identical fiber material, which, however, has not been treated with the at least one agent having at least one, preferably at least two, groups capable of forming a hydrogen bond and the antimicrobial coating composition.

5. The process according to any one of embodiments 1-4, wherein step (2) comprises a step (2a), which is performed after contacting the substrate with the agent having at least one, preferably at least two, groups capable of forming a hydrogen bond and before step (3), wherein step (2a) comprises a step of conditioning the product as obtained from contacting the substrate with an agent having at least one, preferably at least two, groups capable of forming a hydrogen bond.

6. The process according to embodiment 5, wherein step (2a) is a step of tempering the product obtained from contacting the substrate with an agent having at least one, preferably at least two, groups capable of forming a hydrogen bond, at a temperature of at least 30 °C, or
wherein step (2a) is a step of storing the product obtained from contacting the substrate with an agent having at least one, preferably at least two, groups capable of forming a hydrogen bond, at a temperature of 18 °C to less than 30 °C.

7. The process according to embodiment 5 or 6, wherein step (2a) is a step of tempering the product obtained from contacting the substrate with an agent having at least one, preferably at least two, groups capable of forming a hydrogen bond and is performed at a temperature of 30 °C to 120 °C, preferably 40 °C to 110 °C, more preferably 50 °C to 100 °C, more preferably 60 °C to 90 °C, more preferably 70° C to 90 °C, more preferably 75 °C to 85 °C, more preferably about 80 °C.

8. The process according to embodiment 7, wherein step (2a) is performed at a temperature of 75 °C to 85 °C.

9. The process according to any one of embodiments 7 and 8, wherein step (2a) is performed for a duration of 5 min to 90 min, preferably 10 min to 60 min, more preferably 20 min to 40 min, or for longer times up to about 48 hours.

10. The process according to embodiment 9, wherein step (2a) is performed for a duration of 20 min to 40 min.

11. The process according to any one of embodiments 7-10, wherein step (2a) is performed at a temperature of 75 °C to 85 °C and for a duration of 10 to 60 min, preferably 20 min to 40 min.

12. The process according to embodiment 5 or 6, wherein step (2a) is a step of storing the product obtained from contacting the substrate with an agent having at least one, preferably at least two, groups capable of forming a hydrogen bond at a temperature of 18 °C to less than 30 °C, preferably 20 °C to less than 30 °C, more preferably about 20 °C.

13. The process according to embodiment 12, wherein the step of storing the product obtained from contacting the substrate with an agent having at least one, preferably at least two, groups capable of forming a hydrogen bond at a temperature of 18 °C to less than 30 °C, preferably 20 °C to less than 30 °C, more preferably about 20 °C (room temperature), is performed for a duration of at least one week, preferably at least 10 weeks; or wherein said step of storing is performed from 1 week to 40 weeks, preferably from 4 weeks to 30 weeks.

14. The process according to any one of embodiments 5-13, wherein the temperature used in step (2a) is varied at least once over time.

15. The process according to embodiment 14, wherein a temperature gradient is applied.

16. The process according to any one of embodiments 1-15, wherein step (3) is performed more than 10 minutes, preferably more than 15 minutes, more preferably more than 20 minutes, more preferably more than 30 minutes, more preferably more than 40 minutes, more preferably more than 50 minutes, more preferably more than 60 minutes, after the conclusion of step (2), i.e. after the substrate has been contacted with the agent having at least one, preferably at least two, groups capable of forming a hydrogen bond.

17. The process according to any one of embodiments 1-16, wherein step (3) is performed by using slot die, foulard, or kiss coating.

18. The process according to any one of embodiments 1-17, wherein step (4) is performed by subjecting the product of step (3) to a hot air convection oven or to hot plates.

19. The process according to any one of embodiments 1-18, wherein the substrate is a wound dressing substrate.

20. The process according to any one of embodiments 1-19, wherein the substrate is a non-woven substrate.

21. The process according to any one of embodiments 1-20, wherein the substrate further comprises absorbent particles, such as superabsorbent particles.

22. The process according to any one of embodiments 1-21, wherein the substrate has a free swell absorptive capacity, corresponding to the maximum absorptive capacity of the substrate, of at least 1 times its own weight, as measured by EN 13726-1:2002.

23. The process according to any one of embodiments 1-22, wherein the substrate comprises or consists of polyvinyl alcohol, a polysaccharide, polyacrylic acid, polymethacrylic acid, and a copolymer comprising two or more monomers selected from vinyl alcohol, acrylic acid, and methacrylic acid.

24. The process according to any one of embodiments 1-23, wherein the substrate comprises polyvinyl alcohol.

25. The process according to embodiment 24, wherein the substrate consists of polyvinyl alcohol.

26. The process according to any one of embodiments 1-25, wherein the substrate comprises or consists of crosslinked polymeric material.

27. The process according to embodiment 26, wherein the substrate comprises crosslinked polyvinyl alcohol.

28. The process according to embodiment 26, wherein the substrate consists of crosslinked polyvinyl alcohol.

29. The process according to any one of embodiments 1-28, wherein is a web of non-woven polyvinyl alcohol fibers, wherein the polyvinyl alcohol is preferably crosslinked

30. The process according to any one of embodiments 1-29, wherein the agent having at least one, preferably at least two, groups capable of forming a hydrogen bond is provided as such, i.e. without any solvent or adjuvant, or is provided as a compound that is dissolved or distributed in a liquid, in particular in form of a solution, a slurry, an emulsion or the like.

31. The process according to embodiment 30, wherein the liquid is a non-aqueous liquid.

32. The process according to embodiment 31, wherein the liquid comprises ethanol, isopropanol or methanol.

33. The process according to embodiment 31, wherein the liquid is ethanol, isopropanol, methanol, or a mixture thereof.

34. The process according to any one of embodiments 30-33, wherein the concentration of the agent having at least one, preferably at least two, groups capable of forming a hydrogen bond in the liquid is 10 to 100 wt.%, more preferably 20 to 80 wt.%, more preferably 30 to 70 wt.%, more preferably 40 to 60 wt.%, more preferably 45 to 55 wt.%. The concentration is calculated according to the following formula:

$$[c] = [(\text{weight of agent}) / (\text{weight of agent} + \text{weight of liquid})] \times 100,$$

wherein
[c] is the concentration of the agent in wt.%

35. The process according to any one of embodiments 1-34, wherein the agent having at least one, preferably at least two, groups capable of forming a hydrogen bond is selected from the group consisting of polyols, in particular sugar alcohols (sugar polyols), polymeric polyols; polysaccharides, alpha-hydroxy acids; cellulose ethers or cellulose esters; di- or polyisocyanates; polyethers or polyesters.

36. The process according to embodiment 35, wherein the agent having at least one, preferably at least two, groups capable of forming a hydrogen bond is selected from the group consisting of propylene glycol, glycerol, sorbitol, xylitol, maltitol, hexylene glycol, butylene glycol, glyceryl triacetate, polydextrose, lactic acid, panthtothenic acid, hyaluronic acid, sodium-2-pyrrolidone-5- carboxylate ('sodium PCA'), polyethylene glycol (PEG) (also known as polyethylene oxide or polyoxyethylene), polypropylene glycol (PPG), polyvinylpyrrolidone (PVP).

37. The process according to embodiment 35, wherein the agent having at least one, preferably at least two, groups capable of forming a hydrogen bond is a polyol.

38. The process according to embodiment 36 or 37, wherein the agent having at least one, preferably at least two, groups capable of forming a hydrogen bond is glycerol or polyethylene glycol, preferably glycerol.

39. The process according to any one of embodiments 1-38, wherein the silver is present in the form of a silver salt.

40. The process according to embodiment 39, wherein the silver salt is selected from the group consisting of silver sulfate, silver chloride, silver nitrate, silver sulfadiazine, silver carbonate, silver phosphate, silver lactate, silver bromide, silver acetate, silver citrate, silver CMC, silver oxide, preferably silver sulfate.

41. The process according to any one of embodiments 1-40, wherein the silver-containing antimicrobial agent in the antimicrobial coating composition is present in an amount less than 40% w/w.

42. The process according to any one of embodiments 1-41, wherein the silver-containing antimicrobial agent in the substrate is present in an amount less than 30 mg/cm$^2$.

43. The process according to any one of embodiments 1-42, wherein the one or more polymers in the antimicrobial coating composition are selected from the group consisting of cellulosic polymers, neutral poly(meth)acrylate esters, polyvinylpyrrolidone, polyvinylpolypyrrolidone, and combinations thereof.

44. The process according to embodiment 43, wherein the one or more polymers in the coating composition is selected from cellulosic polymers, preferably from hydroxypropylmethylcellulose (HPMC), hydroxypropylcellulose (HPC), methylcellulose (MC), ethylcellulose (EC), and combinations thereof.

45. The process according to embodiment 44, wherein the one or more polymers in the coating composition is HPC.

46. The process according to any one of embodiments 1-45, wherein each of the one or more polymers in the antimicrobial coating composition is present in an amount less than 30% w/w.

47. The process according to any one of embodiments 1-46, wherein the non-aqueous solvent comprises a polar protic solvent, wherein the polar protic solvent preferably is an alcohol, preferably a C$_{1-4}$ alkyl alcohol.

48. The process according to any one of embodiments 1-46, wherein the non-aqueous solvent is a polar protic solvent, wherein the polar protic solvent preferably is an alcohol, preferably a C$_{1-4}$ alkyl alcohol.

49. The process according to embodiment 47 or 48, wherein the polar protic solvent comprises methanol, ethanol, n-propanol, isopropanol, n-butanol, or s-butanol, preferably ethanol.

50. The process according to any one of embodiments 47-49, wherein the polar protic solvent is methanol, ethanol, *n*-propanol, isopropanol, *n*-butanol, or *s*-butanol, preferably ethanol.

51. The process according to any one of embodiments 1-45, wherein the antimicrobial coating composition comprises less than 50% w/w of water, preferably less than 40% w/w of water, more preferably less than 30% w/w of water, even more preferably less than 20% w/w of water.

52. The process according to embodiment 51, wherein the antimicrobial coating composition comprises from 0% to 40% w/w of water, preferably from 1% to 40% w/w of water further preferably from 1 % to 20% w/w of water.

53. The process according to embodiment 51 or 52, wherein the antimicrobial coating composition comprises no water or only trace amounts of water.

54. Fiber material obtained or obtainable by the process of any one of embodiments 1-53.

55. Wound dressing, comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows a change in the L value Δ L as defined in subsection 6.2.3 of ASTM D2244-16 of 10% or less, preferably 5% or less, further preferably 3% or less, over a time period of ten weeks, wherein the wound dressing is exposed to a temperature of 30°C in ambient air, at a humidity of 75%, over the entire ten week period, wherein the wound dressing has been sterilized prior to the 10 week period and wherein the Δ L value is measured as an average of at least ten measurements on 5 different spots on a 10 cm times 10 cm sample of the wound dressing.

56. Wound dressing, comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows less than 0.5, preferably less than 0.1, further preferably no spots or speckles, as visible to the eye, in 10 different segments of an area of 1cm$^2$, respectively, as present on a wound dressing of an overall area of 10cm$^2$, wherein the number of spots or speckles as counted in all 10 segments is divided by 10 and wherein the spots or speckles as visible to the eye are determined after the lapse of a time period of ten weeks in which the wound dressing is exposed to a temperature of 30°C in ambient air, at a humidity of 75%, over the entire ten week period, wherein the wound dressing has been sterilized prior to the 10 week period.

57. The wound dressing according to embodiment 55 or embodiment 56, wherein the substrate, the at least one antimicrobial agent, the at least one polymer, and the agent having at least one, preferably at least two, groups capable of forming a hydrogen bond are defined as set out in any one of embodiments 1-53 described above.

**EXAMPLES**

The following materials were used in the examples of the present application:

***Example 1. Preparation of fiber material according to the present invention.***

A mixture of 117 g glycerol and 400 g ethanol was prepared by mixing and stirring. A PVA nonwoven substrate (commercially available Exufiber 20x30 cm substrate, REF 603303-0) was coated with the mixture on both sides thereof (100 grams per square meter ("gsm") of mixture per side of substrate, i.e. total coating weight 200 gsm). The so obtained coated substrate was heated on a hot plate at 80 °C for 2 minutes in order to remove the ethanol. The so obtained material was heated in a convection oven at 80 °C for a further 30 minutes. After that, the substrate was removed from the oven and kept at room temperature until the temperature of the substrate decreased to room temperature.
In parallel, an antimicrobial coating composition was prepared by mixing 600 g of ethanol, 69 g water, 12 g of silver sulfate, and 8 g of hydroxypropyl cellulose ("HPC").
The antimicrobial coating composition was coated onto the glycerol-containing substrate. The coated substrate was then heated on a hot plate at 80 °C for 2 minutes in order to remove the ethanol.
The so prepared coated substrate had a soft touch and did not show any discoloration.
The coated substrate was subsequently sterilized twice using ethylene oxide sterilization.

***Comparative Example 1 (labeled as sample "C" in Figure 2, lowermost curve)***

A mixture of 105 g glycerol, 8.1 g silver sulfate, 5.4 g HPC, 47 g water, and 300 g ethanol was prepared by mixing and stirring. A PVA nonwoven substrate (commercially available Exufiber 20x30 cm substrate, REF 603303-0) was coated with the mixture on both side thereof (100 gsm of mixture per side of substrate, i.e. total coating weight 200 gsm) and the so obtained coated substrate was heated on a hot plate at 80 °C for 2 minutes in order to remove the ethanol. The so obtained material was heated in a convection oven at 80 °C for a further 30 minutes.
The coated substrate was subsequently sterilized twice using ethylene oxide sterilization.
The so prepared coated substrate had a soft touch but showed severe discoloration over time, as can be seen in Figure 2, which shows the evolution of discoloration, as measured by the $\Delta$ L value in the (L*a*b) color space over a time period of 17 weeks, wherein "A" (uppermost curve) is an already commercialized dressing based on fibers that are coated with silver, but not with an agent capable of forming a hydrogen bond (glycerol in the Example according to the present invention). This commercialized dressing shows basically no discoloration. In Figure 2, "C" (lowermost curve) is the reference sample of Comparative Example 1, wherein glycerol and silver are coated *together* onto the fiber and then subjected to a heat treatment to remove the alcohol. "E" (second curve that essentially tracks the uppermost curve) is then the sample in accordance with the present invention, wherein the antimicrobial composition comprising silver is coated on fibers that have already been coated with glycerol in a previous step. As can be seen from Figure 2, the fibers as coated with glycerol and silver in accordance with the present invention show almost as little discoloration as the already commercialized fiber dressing that is only coated with silver. The advantage of the inventive wound dressing "E" over the already commercialized dressing "A" is the improved wet tensile strength due to the pre-treatment with the agent capable of forming hydrogen bonds. While sample "C" also may have an increased wet tensile strength, said sample shows significant discoloration and the presence of spots and speckles (see Figure 3) that is believed to be due to instable coating if silver is coated onto the fiber together with the agent capable of forming a hydrogen bond.

**Claims**

1.    A process for making a modified fiber material, wherein the process comprises the following steps:

(1) providing a substrate comprising or consisting of absorbent fibers;
(2) contacting the substrate with an agent having at least one, preferably at least two, groups capable of forming a hydrogen bond;
(3) contacting the product of step (2) with an antimicrobial coating composition comprising at least one silver-containing antimicrobial agent and one or more polymers in a solvent system that comprises a non-aqueous solvent.

(4) drying the product of step (2), or the product of step (3), or both.

2.   The process of claim 1, wherein the modified fiber material is comprised in a wound dressing.

3.   The process of claim 1 or 2, wherein the product of step (4) has a wet tensile strength of at least 0.2 N/ 2cm, wherein said wet tensile strength is determined as set out in the description., optionally wherein the wet tensile strength of the product of step (4) is increased by at least 5%, preferably at least 10% and further preferably by at least 15%, compared to an otherwise identical fiber material, which, however, has not been treated with the at least one agent having at least one, preferably at least two, groups capable of forming a hydrogen bond and the antimicrobial coating composition.

4.   The process according to any one of claims 1-3, wherein step (2) comprises a step (2a), which is performed after contacting the substrate with the agent having at least one, preferably at least two, groups capable of forming a hydrogen bond and before step (3), wherein step (2a) comprises a step of conditioning the product as obtained from contacting the substrate with an agent having at least one, preferably at least two, groups capable of forming a hydrogen bond.

5.   The process according to claim 4, wherein step (2a) is a step of tempering the product obtained from contacting the substrate with an agent having at least one, preferably at least two, groups capable of forming a hydrogen bond, at a temperature of at least 30 °C, or
wherein step (2a) is a step of storing the product obtained from contacting the substrate with an agent having at least one, preferably at least two, groups capable of forming a hydrogen bond, at a temperature of 18 °C to less than 30 °C.

6.   The process according to any one of claims 1-5, wherein the substrate has a free swell absorptive capacity, corresponding to the maximum absorptive capacity of the substrate, of at least 1 times its own weight, as measured by EN 13726-1:2002, and/or
wherein the substrate comprises or consists of polyvinyl alcohol, a polysaccharide, polyacrylic acid, polymethacrylic acid, and a copolymer comprising two or more monomers selected from vinyl alcohol, acrylic acid, and methacrylic acid.

7.   The process according to any one of claims 1-6, wherein the agent having at least one, preferably at least two, groups capable of forming a hydrogen bond is provided as such, i.e. without any solvent or adjuvant, or is provided as a compound that is dissolved or distributed in a liquid, in particular in form of a solution, a slurry, an emulsion or the like.

8.   The process according to claim 7, wherein the concentration of the agent having at least one, preferably at least two, groups capable of forming a hydrogen bond in the liquid is 10 to 100 wt.%, more preferably 20 to 80 wt.%, more preferably 30 to 70 wt.%, more preferably 40 to 60 wt.%, more preferably 45 to 55 wt.%. The concentration is calculated according to the following formula:

$$[c] = [(\text{weight of agent}) / (\text{weight of agent} + \text{weight of liquid})] \times 100,$$

wherein
[c] is the concentration of the agent in wt.%

9.   The process according to any one of claims 1-8, wherein the agent having at least one, preferably at least two, groups capable of forming a hydrogen bond is selected from the group consisting of polyols, in particular sugar alcohols (sugar polyols), polymeric polyols; polysaccharides, alpha-hydroxy acids; cellulose ethers or cellulose esters; di- or polyisocyanates; polyethers or polyesters, optionally:
wherein the agent having at least one, preferably at least two, groups capable of forming a hydrogen bond is selected from the group consisting of propylene glycol, glycerol, sorbitol, xylitol, maltitol, hexylene glycol, butylene glycol, glyceryl triacetate, polydextrose, lactic acid, panthtothenic acid, hyaluronic acid, sodium-2-pyrrolidone-5- carboxylate ('sodium PCA'), polyethylene glycol (PEG) (also known as polyethylene oxide or polyoxyethylene), polypropylene glycol (PPG), polyvinylpyrrolidone (PVP).

10.  The process according to any one of claims 1-9, wherein the silver is present in the form of a silver salt, optionally:

wherein the silver salt is selected from the group consisting of silver sulfate, silver chloride, silver nitrate, silver sulfadiazine, silver carbonate, silver phosphate, silver lactate, silver bromide, silver acetate, silver citrate, silver CMC, silver oxide, preferably silver sulfate.

11. The process according to any one of claims 1-10, wherein the one or more polymers in the antimicrobial coating composition are selected from the group consisting of cellulosic polymers, neutral poly(meth)acrylate esters, polyvinylpyrrolidone, polyvinylpolypyrrolidone, and combinations thereof, optionally:
wherein the one or more polymers in the coating composition is selected from cellulosic polymers, preferably from hydroxypropylmethylcellulose (HPMC), hydroxypropylcellulose (HPC), methylcellulose (MC), ethylcellulose (EC), and combinations thereof.

12. The process according to any one of claims 1-11, wherein the non-aqueous solvent comprises or is a polar protic solvent, wherein the polar protic solvent preferably is an alcohol, preferably a $C_{1-4}$ alkyl alcohol.

13. The process according to any one of claims 1-12, wherein the antimicrobial coating composition comprises less than 50% w/w of water, preferably less than 40% w/w of water, more preferably less than 30% w/w of water, even more preferably less than 20% w/w of water.

14. Fiber material obtained or obtainable by the process of any one of claims 1-13.

15. Wound dressing, comprising (i) a substrate comprising or consisting of absorbent fibers, (ii) at least one silver-containing antimicrobial agent, (iii) at least one polymer, and (iv) an agent having at least one, preferably at least two, groups capable of forming a hydrogen bond, wherein the wound dressing shows a change in the L value $\Delta$ L as defined in subsection 6.2.3 of ASTM D2244-16 of 10% or less, preferably 5% or less, further preferably 3% or less, over a time period of ten weeks, wherein the wound dressing is exposed to a temperature of 30°C in ambient air, at a humidity of 75%, over the entire ten week period, wherein the wound dressing has been sterilized prior to the 10 week period and wherein the $\Delta$ L value is measured as an average of at least ten measurements on 5 different spots on a 10 cm times 10 cm sample of the wound dressing.

16. The wound dressing according to claim 15, wherein the substrate, the at least one antimicrobial agent, the at least one polymer, and the agent having at least one, preferably at least two, groups capable of forming a hydrogen bond are defined as set out in any one of claims 1-14.

Figure 1

Figure 2

Figure 3

Europäisches
Patentamt

European
Patent Office

Office européen
des brevets

# EUROPEAN SEARCH REPORT

**Application Number**

EP 21 16 4631

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/380880 A1 (CARLSSON ERIK [SE]) 19 December 2019 (2019-12-19) * table 1 * * paragraph [0044] * * examples 1-3 * * figure 1 * * paragraph [0106] * ----- | 1-16 | INV. A61L15/46 |
| X | US 5 326 567 A (CAPELLI CHRISTOPHER C [US]) 5 July 1994 (1994-07-05) * examples 1-16 * ----- | 15,16 | |

**TECHNICAL FIELDS SEARCHED (IPC)**

A61L

The present search report has been drawn up for all claims

| Place of search | Date of completion of the search | Examiner |
|---|---|---|
| The Hague | 2 September 2021 | Siebum, Bastiaan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 16 4631

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2021

| Patent document cited in search report | | | Publication date | Patent family member(s) | | | Publication date |
|---|---|---|---|---|---|---|---|
| US 2019380880 | A1 | | 19-12-2019 | AU | 2018212384 | A1 | 11-07-2019 |
| | | | | BR | 112019015285 | A2 | 03-03-2020 |
| | | | | CA | 3049506 | A1 | 02-08-2018 |
| | | | | CN | 110225735 | A | 10-09-2019 |
| | | | | EP | 3354242 | A1 | 01-08-2018 |
| | | | | EP | 3573587 | A1 | 04-12-2019 |
| | | | | JP | 2020507687 | A | 12-03-2020 |
| | | | | KR | 20190142316 | A | 26-12-2019 |
| | | | | US | 2019380880 | A1 | 19-12-2019 |
| | | | | WO | 2018137979 | A1 | 02-08-2018 |
| US 5326567 | A | | 05-07-1994 | AT | 181822 | T | 15-07-1999 |
| | | | | AU | 656384 | B2 | 02-02-1995 |
| | | | | BR | 9205879 | A | 05-07-1994 |
| | | | | CA | 2108008 | A1 | 11-10-1992 |
| | | | | DE | 69229548 | T2 | 17-02-2000 |
| | | | | EP | 0580803 | A1 | 02-02-1994 |
| | | | | JP | H06506694 | A | 28-07-1994 |
| | | | | US | 5326567 | A | 05-07-1994 |
| | | | | WO | 9218098 | A1 | 29-10-1992 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018137979 A
- WO 2017016974 A
- US 20130323195 A
- US 20130274415 A

**Non-patent literature cited in the description**

- hydrogen bond. *Pure Appl. Chem,* 2011, vol. 83 (8), 1637-1641